# EUROPÄISCHE PATENTSCHRIFT

(11) **EP 0 767 904 B1**
(45) Veröffentlichungstag und Bekanntmachung des Hinweises auf die Patenterteilung: **13.05.1998**
(21) Anmeldenummer: 95924883.2
(22) Anmeldetag: 20.06.1995
(51) Int. Cl.: G01N 21/86, G01N 21/89

(54) **VORRICHTUNG ZUR BESTIMMUNG VON MATERIALEIGENSCHAFTEN**
DEVICE FOR DETERMINING PROPERTIES OF MATERIALS
DISPOSITIF DE DETERMINATION DES PROPRIETES DE MATERIAUX

(30) Priorität: 30.06.1994 DE 4422861
(43) Veröffentlichungstag der Anmeldung: 16.04.1997
(73) Patentinhaber: HONEYWELL AG, 63067 Offenbach (DE)
(72) Erfinder: SYRE, Hans-Richard, D-56566 Neuwied (DE)
(74) Vertreter: Herzbach, Dieter, Dipl.-Ing.
(86) Internationale Anmeldenummer: EP9502386
(87) Internationale Veröffentlichungsnummer: WO9600894

(56) Entgegenhaltungen:
- US-A- 3 906 232
- US-A- 4 224 513
- US-A- 5 073 712

## Beschreibung

Die vorliegende Erfindung bezieht sich auf eine Vorrichtung zur Bestimmung von Materialeigenschaften nach dem Gattungsbegriff des Patentanspruches 1.

In diesem Zusammenhang ist es bereits bekannt, Materialeigenschaften, wie z.B. die Feuchte und das Basisgewicht von bewegten Papierbahnen zu messen, indem man die Papierbahn beleuchtet und im Durchlicht oder Reflexlicht bestimmte Infrarot-Absorptionsbande über Infrarot-Filter ermittelt und auswertet. Um die ganze Breite der Papierbahn hinsichtlich ihrer Eigenschaften zu erfassen, ist entweder ein quer zu der Papierbahn beweglicher Meßwagen vorgesehen oder es wird in einer über der Papierbahn angeordneten stationären Meßvorrichtung ein beweglicher Abtast-Lichtfleck erzeugt und die im reflektierten Licht enthaltenen Infrarot-Absorptionsbanden ausgewertet. Die zuletzt genannte Meßvorrichtung kann z.B. der US-A-5 073 712 entnommen werden. Zur Steigerung der Meßgenauigkeit sind bei dieser bekannten Anordnung auch noch Kalibriermuster innerhalb der stationären Meßvorrichtung und Kompensationsmuster seitlich von der bewegten Materialbahn angeordnet.

Aus der EP-0 390 623 A2 ist ferner ein optisches System zur Feststellung von Eigenschaften eines bewegten blattförmigen Materials bekannt, bei dem zu beiden Seiten der Materialbahn optische Fasern angeordnet sind, die sich über die Breite der Materialbahn erstrecken und die Licht von einer Lichtquelle zu der Materialbahn und von der Materialbahn zu Empfängern leiten.

Ausgehend von diesem Stand der Technik ist es die Aufgabe der vorliegenden Erfindung, eine Meßvorrichtung anzugeben, die in einfacher Weise die ganzflächige Erfassung von Materialeigenschaften gestattet. Die Lösung dieser Aufgabe gelingt gemäß den kennzeichnenden .Merkmalen des Patentanspruches 1. Weitere vorteilhafte Ausgestaltungen der erfindungsgemäßen Vorrichtung sind den abhängigen Ansprüchen entnehmbar.

Anhand der Figuren der beiliegenden Zeichnung sei im folgenden die Vorrichtung gemäß der Erfindung näher beschrieben. Es zeigen:
Fig. 1 eine Prinzipdarstellung der gesamten Vorrichtung;
Fig. 2 eine Darstellung der verwendeten Filteranordnung;
Fig. 3 das Auswerteschema eines Teil-Kamerabildes; und
Fig. 4 das Prinzip der Gesamtauswertung.

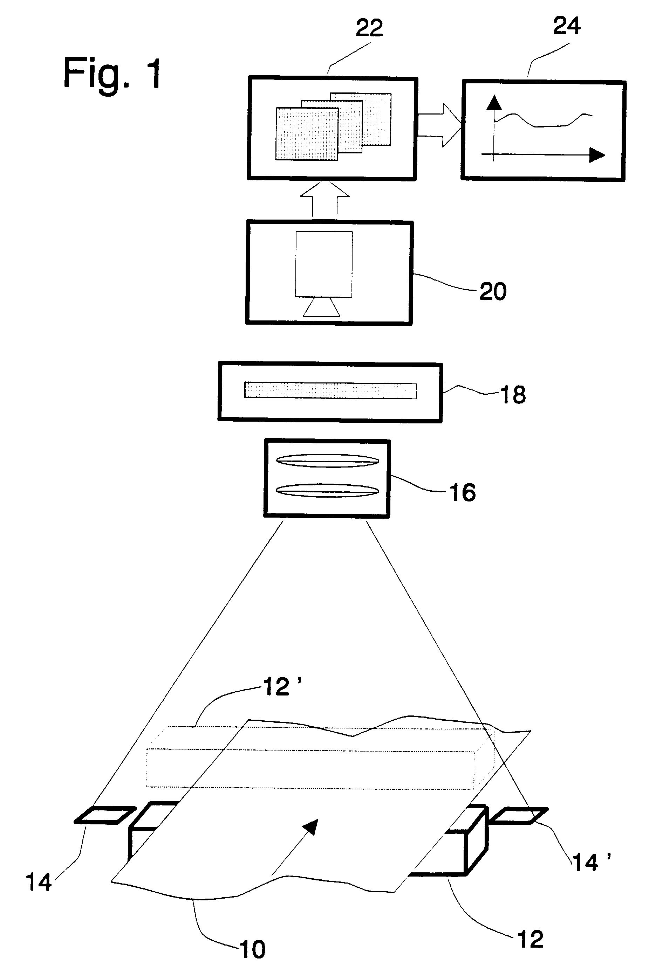

Gemäß Fig. 1 wird eine senkrecht zur Zeichenebene verlaufende Materialbahn 10, die das Meßgut darstellt, durch eine linienförmige oder rechteckige Lichtquelle 12, die quer zur Materialbahn 10 angeordnet ist, beleuchtet. Das Licht umfaßt vorzugsweise weißes Licht; kann aber auch durch einen Infrarot-Strahler erzeugt werden. Für ein mehr oder minder lichtundurchlässiges Material 10 erfolgt die Messung im reflektierten Licht und die Lichtquelle 12' ist daher oberhalb der Materialbahn 10 angeordnet. Bei der Messung von transparentem Material befindet sich die Lichtquelle 12 unterhalb der Materialbahn 10.

Zu beiden Seiten und neben der Materialbahn 10 ist jeweils eine Referenzlichtquelle 14, 14' angeordnet, die ein dem Meßgut ähnliches oder gleiches Spektrum besitzt, was durch die Vorschaltung einer entsprechenden Kombination von Infrarot-Filtern vor die Lichtquellen erzielt wird. Es kann aber auch eine Referenzprobe des jeweiligen Meßgutes als Kompensationsmuster eingesetzt werden. Bei oft wechselnden Meßaufgaben sind Vorrichtungen erforderlich, bei der einzelne Filter hinzugefügt oder herausgenommen werden können, um die Referenzlichtquelle optimal auszulegen.

Das durch die Materialbahn 10 vorgegebene Meßgut umfaßt bahnförmige, blattförmige Güter, wie Papier, beschichteten Karton, lackierte Bleche und durchsichtige oder opake Folien aus Kunststoff oder Zellulose.

Der durch die Lichtquelle 12, 12' beleuchtete Streifen der Materialbahn 10 wird über eine für Infrarotlicht taugliche Optik 16 über eine Infrarot-Filteranordnung 18 auf eine Infrarot-Kamera 20 abgebildet.

Die Optik 16 dient der Abbildung des Lichtstreifens und umfaßt ein Linsensystem, wobei für die Linsen vorzugsweise Gläser, die durch ihre Zusammensetzung infrarottauglich sind, weiterhin Materialien wie beispielsweise Germanium, Silicium, Zinksulfid oder Saphir verwendet werden. Die Linsen können entweder rotationssymmetrisch sein, es können aber auch anamorphotische Linsensysteme eingesetzt werden.

Das Linsensystem kann ohne Verlust der Funktion durch ein System von Hohlspiegeln, Parabolspiegeln, Zylinderspiegeln oder torischen Spiegeln ersetzt werden.

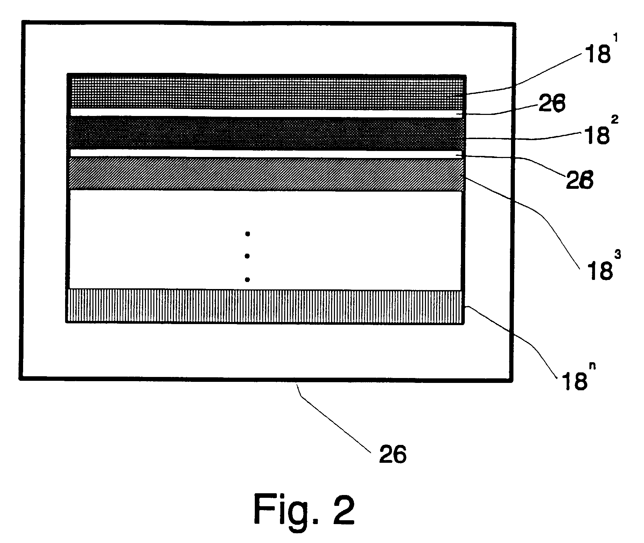

Die Infrarot-Filteranordnung 18 besteht aus einem Array von rechteckigen Einzel-Filterstreifen 18¹ bis 18ⁿ, deren Längsseiten quer zur Laufrichtung des Meßgutes angeordnet sind. Die Einzel-Filterstreifen sind vorzugsweise Interferenzfilter, deren Zentralwellenlänge, Bandbreite und Transmissionsgrad auf das jeweilige Meßproblem abgestimmt sind. Die Einzelfilter 18¹ bis 18ⁿ sind in einen Rahmen 26 streifenförmig eingeklebt und zwischen den Einzelfeldern sind optisch dichte Trennwände 28 angeordnet, um ein optisches Übersprechen zwischen den Einzelfiltern zu unterbinden, wie dies aus Fig. 2 ersichtlich ist.

Die Infrarot-Kamera 20 kann sämtliche Arten von infrarottauglichen Bildaufnehmern umfassen, d.h. Bildaufnehmer für den Wellenlängenbereich von 0,7 µm bis 18 µm. Eine solche Kamera kann aus einem Array von infrarotempfindlichen Detektoren bestehen, die eine Bilderzeugung ermöglichen und die als zweidimensionale bolometrische Kameras bekannt sind. Es ist auch möglich, einen Bildwandler einzusetzen, der Licht im nahen Infrarotbereich in sichtbares Licht umwandelt, so daß eine Kamera für sichtbares Licht zum Einsatz kommen kann.

Die Kamera 20 ist an einen Rechner 22 angeschlossen, der seinerseits mit einer Anzeigeeinheit 24 verbunden ist. Die Bildverarbeitung in dem Rechner 22 umfaßt die gesamte Hard- und Software zur Aufbereitung des ankommenden analogen zeit seriellen Signals zu einem Materialeigenschaftsprofil. Zu diesem Zweck wird das Bildsignal zunächst digitalisiert und entsprechend der Bildelementposition in definierten Speicherzellen abgelegt. Entsprechende hardwaremäßige Implementierungen sind als Bildeinzugseinheit (Framegrabber) im Stand der Technik verfügbar.

Die im Rechner implementierte Software hat die Aufgabe, die Bilder der einzelnen Infrarot-Filterstreifen zu Absorptionsprofilen zu verarbeiten.

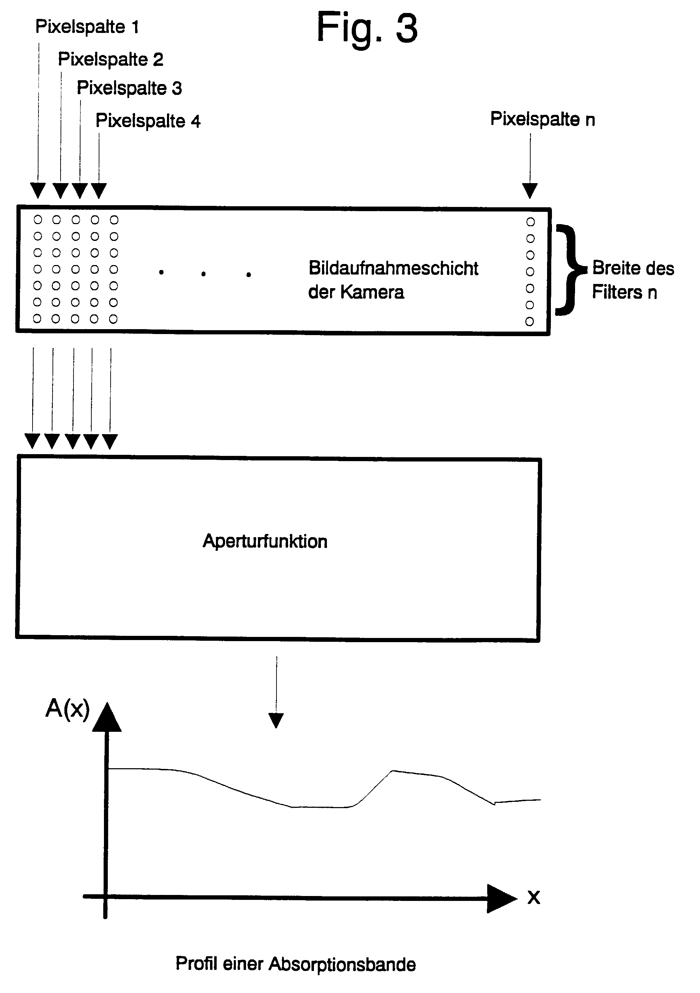

Fig. 3 zeigt die Verarbeitung eines Einzel-Filterstreifens, wobei die zur Filterlängsrichtung senkrecht stehenden Pixelreihen, die durch einen Einzel-Filterstreifen in der Kamera erzeugt werden, bezüglich ihrer Werte, vorzugsweise gemittelt werden und dieser gemittelte Wert in die zugehörige Profilposition geschrieben wird. Bei der Berechnung des Profils sind Aperturfunktionen anwendbar, die die rechts und links stehenden Pixelelemente gewichtet in die Berechnung mit einbeziehen. Als Ergebnis dieser Verarbeitung ergibt sich ein Profil einer bestimmten Absorptionsbande, wie es in der unteren Hälfte von Fig. 3 dargestellt ist und wobei die Amplitude A der Absorptionsbande über der Breite x des Meßgutes aufgetragen ist.

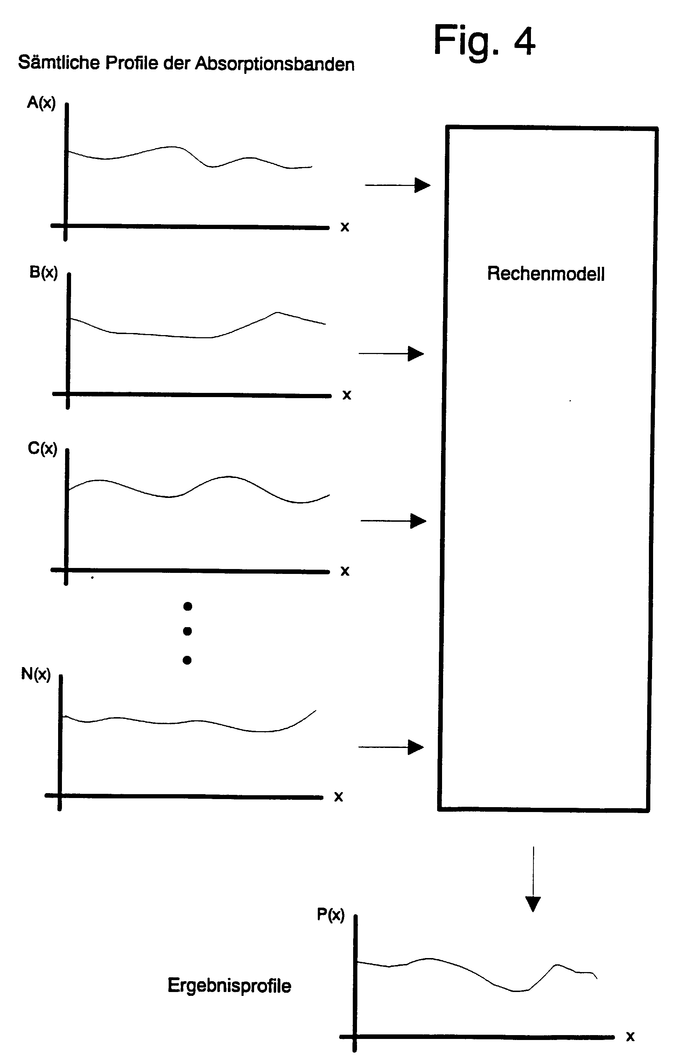

Der nächste Schritt besteht in der Berechnung der eigentlichen Materialeigenschaft aus den Absorptionsprofilen, wobei z.B. profilpositionsweise ein n-Tupel von Werten als Argumente in einen Algorithmus eingebracht werden. Dieser Algorithmus ist in dem Rechner 22 mit einer vorgegebenen Software verwirklicht, wobei die einzelnen Absorptionsprofile A(x) bis N(x) zu einem Ergebnisprofil P(x) umgerechnet werden, wie dies in Fig. 4 dargestellt ist.

## Patentansprüche

1. Vorrichtung zur Bestimmung von Materialeigenschaften von bewegtem blattförmigem Material, insbesondere von bewegten Papierbahnen, mit einer sich quer zu dem bewegten Material erstreckenden Beleuchtungseinrichtung für die Beleuchtung des Materials, mit einer Filter- und Detektoreinrichtung zur Erfassung von Absorptionsbanden innerhalb des von dem Material beeinflußten Lichts und mit einer die Absorptionsbanden auswertenden Einrichtung zur Bestimmung der Materialeigenschaften, **gekennzeichnet durch** folgende Merkmale:
a) das blattförmige Material (10) wird bandformig beleuchtet;
b) es ist eine aus Einzelfilterstreifen (18¹-18ⁿ) bestehende zweidimensionale Filteranordnung (18) angeordnet, deren Einzelfilterstreifen sich quer zur Bewegungsrichtung des Materials (10) erstrecken; und
c) eine Flächenkamera (20) dient der zeilenweisen Erfassung der von den Einzelfilterstreifen (18¹-18ⁿ) gelieferten Absorptionsbanden, die durch die Auswerteeinrichtung (22) in ensprechende Materialprofile umgerechnet werden.

2. Vorrichtung nach Anspruch 1, **dadurch gekennzeichnet**, daß die bandförmige Beleuchtung durch eine bandförmige Lichtquelle (12,12') aus weißem Licht erfolgt, daß die Filteranordnung (18) aus Infrarot-Streifenfiltern (18¹-18ⁿ) besteht, auf die das streifenförmig beleuchtete Material über eine Infrarot-Optik (16) abgebildet wird und daß die Kamera (20) eine Infrarot-Kamera ist.

3. Vorrichtung nach Anspruch 2, **dadurch gekennzeichnet**, daß seitlich von dem blattförmigen Material (10) jeweils Referenzlichtquellen (14,14') angeordnet sind, die ebenfalls über die Infrarot-Optik (16) und die Infrarot-Filteranordnung (18) auf die Infrarot-Kamera (20) abgebildet werden.

4. Vorrichtung nach Anspruch 3, **dadurch gekennzeichnet**, daß die Signale der Infrarot-Kamera (20) digitalisiert, gespeichert und einer Datenverarbeitung in einem Rechner (22) zugeführt werden.

5. Vorrichtung nach Anspruch 2, **dadurch gekennzeichnet**, daß die Infrarot-Streifenfilter (18¹-18ⁿ) unter Zwischenfügung von optisch dichten Trennwänden (28) in einen Rahmen (26) eingeklebt sind.

6. Vorrichtung nach Anspruch 2, **dadurch gekennzeichnet**, daß die Infrarot-Kamera (20) eine zweidimensionale bolometrische Kamera ist.

7. Vorrichtung nach Anspruch 5, **dadurch gekennzeichnet**, daß die Infrarot-Streifenfilter (18¹-18ⁿ) durch Interferenzfilter vorgegeben sind.

8. Vorrichtung nach Anspruch 3, **dadurch gekennzeichnet,** daß den Referenzlichtquellen (14,14') Kombinationen von Infrarot-Filtern vorgeschaltet sind, um ein an das Meßgut (10) angepaßtes Spektrum vorzugeben.

## Claims

1. Device for determining properties of materials of a moving laminar-shaped material, in particular of moving paper webs, comprising an illuminating device extending transverse to the moving material and for illuminating said material, a filter and detector device for sensing absorption bands of light being influenced inside of said material and a device for evaluating said absorption bands in order to determine properties of said material, **characterized by** the following features:
a) the laminar-shaped material (10) is illuminated along a strip;
b) a two-dimensional filter device (18) is provided which consists of single filter strips (18¹-18ⁿ) with the filter strips extending transverse to the guide motion of the material (10); and
c) a flat camera (20) serves to sense line-by-line the absorption bands provided by said single filter strips (18¹-18ⁿ), which are converted by an evaluation device (22) into corresponding material profiles.

2. Device according to claim 1, **characterized in that** the strip-shaped illumination is effected by a strip-shaped light source (12, 12') of white light, that the filter device (18) consists of strip-shaped infrared filters (18¹-18ⁿ), onto which the material illuminated in strips is imaged via an infrared optics (10) and that the camera (20) is an infrared camera.

3. Device according to claim 2, **characterized in that** lateral to the laminar-shaped material (10) reference light sources (14, 14') each are located which are also imaged onto the infrared camera (20) via the infrared optics (16) and the infrared filter device (18).

4. Device according to claim 3, **characterized in that** the signals ofthe infrared camera (20) are digitized, memorized and are fed to a computer for data processing.

5. Device according to claim 2, **characterized in that** the strip-shaped infrared filters (18¹-18ⁿ) are gummed into a frame (26) with an intermediate arrangement of optical light separating walls (28).

6. Device according to claim 2, **characterized in that** the infrared camera (20) is a two-dimensional bolometric camera.

7. Device according to claim 5, **characterized in that** the strip-shaped infrared filters (18¹-18ⁿ) are provided by interference filters.

8. Device according to claim 3, **characterized in that** a combination of infrared filters is arranged in front of the reference light sources (14, 14') in order to match the spectrum to the material (10) under test.

## Revendications

1. Dispositif pour déterminer des caractéristiques d'un matériau en forme de feuilles mobile, notamment de bandes de papier mobiles, comportant un dispositif d'éclairement qui s'étend transversalement par rapport au matériau mobile et sert à éclairer le matériau, un dispositif de filtrage et de détection pour détecter des bandes d'absorption à l'intérieur de la lumière influencée par le matériau, et un dispositif qui exploite les bandes d'absorption et sert à déterminer les caractéristiques du matériau, caractérisé par les caractéristiques suivantes :
a) le matériau en forme de feuilles (10) est soumis à un éclairement en forme de bande ;
b) il est prévu un dispositif de filtrage bidimensionnel (18), qui est constitué par des bandes individuelles de filtrage (18¹-18ⁿ) et dont les bandes individuelles de filtrage s'étendent transversalement par rapport à la direction de déplacement du matériau (10) ; et
c) une caméra de surface (20) sert à réaliser la détection ligne par ligne des bandes d'absorption délivrées par les bandes individuelles de filtrage (18¹-18ⁿ) et qui sont converties par le dispositif d'exploitation (22) en des profils correspondants du matériau.

2. Dispositif selon la revendication 1, caractérisé en ce que l'éclairement en forme de bande est réalisé au moyen d'une source de lumière en forme de bande (12, 12') délivrant une lumière blanche, que le dispositif de filtrage (18) est constitué par des filtres en forme de bandes pour l'infrarouge (18¹-18ⁿ), sur lesquels l'image du matériau éclairé en forme de bande est formée par l'intermédiaire d'un système optique à infrarouge (16), et que la caméra (20) est une caméra infrarouge.

3. Dispositif selon la revendication 2, caractérisé en ce que des sources respectives de lumière de référence (14, 14'), dont l'image est formée sur la caméra infrarouge (20) également par l'intermédiaire du système optique à infrarouge (16) et du dispositif de filtrage à infrarouge (18), sont disposées latéralement par rapport au matériau en forme de feuilles (10).

4. Dispositif selon la revendication 3, caractérisé en ce que les signaux de la caméra infrarouge (20) sont numérisés, mémorisés et envoyés à un dispositif de traitement de données dans un ordinateur (22).

5. Dispositif selon la revendication 2, caractérisé en ce que les filtres en forme de bandes à infrarouge (18¹-18ⁿ) sont collés dans un cadre (26) moyennant l'interposition de parois de séparation (28) étanches du point de vue optique.

6. Dispositif selon la revendication 2, caractérisé en ce que la caméra à infrarouge (20) est une caméra bolométrique bidimensionnelle.

7. Dispositif selon la revendication 5, caractérisé en ce que les filtres en forme de bandes à infrarouge (18¹-18ⁿ) sont prédéterminés par des filtres interférentiels.

8. Dispositif selon la revendication 3, caractérisé en ce que des combinaisons de filtres à infrarouge sont disposées devant les sources de lumière de référence (14, 14') de manière à prédéterminer un spectre adapté au matériau à mesurer (10).
